# EUROPEAN PATENT APPLICATION

(11) **EP 4 039 264 A1**
(43) Date of publication of application: **10.08.2022**
(21) Application number: 21190700.1
(22) Date of filing: 10.08.2021
(51) Int. Cl.: A61K 38/08, A61K 38/10, A61P 25/16, A61P 25/28

(54) **ORAL PEPTIDE ANTAGONISTS OF MULTIPLE CHEMOKINE RECEPTORS FOR REVERSING LOSS OF SYNAPSES AND DENDRITIC SPINES**

(30) Priority: 08.02.2021 US 202117170790
(71) Applicant: Creative Bio-Peptides Inc., Potomac MD 20854 (US)
(72) Inventor: RUFF, Michael R., Potomac, 20854 (US); ROSNER, Gilah, Potomac, 20854 (US)
(74) Representative: Avidity IP

(57) **Abstract**

The present invention relates, broadly, to the improvement of cognition by methods to shift neurons from a state or condition of loss of synapses and dendritic spines to one of formation/regeneration of synapses and dendritic spines. A novel mechanism appears to control a neuronal stress response and improve memory and learning by rebalancing synapse and dendritic spine dynamics to favor their formation. Methods are disclosed to enhance the number of functioning synapses and dendritic spines which is expected to improve overall cognitive function.

## Description

The present invention relates, broadly to compositions and compounds that enter the central nervous system to engage neuronal chemokine receptors to promote neuronal remodeling and synapse and dendritic spine formation. In particular, chemokine receptor antagonists have been found to promote memory and learning in normal animals, and in conditions of brain injury promote synapse and dendritic spine formation for recovery of cognitive and motor abilities. The invention promotes formation of synapses and dendritic spines and is expected to enhance memory and learning in normal individuals and in those who experience loss of synapses, for example due to aging, or disease condition where synapse and dendritic spine loss is accelerated leading to cognitive or motor functional impairments.

### BACKGROUND

Improved cognition and mental function is a commonly sought goal. Regulation of protein translation, protein degradation, cytoskeletal dynamics, extracellular matrix interactions, second messenger signaling, and neurotransmitter receptor trafficking and function are all components of synaptic remodeling essential for cognition. Selective targeting of specific components in these processes can serve as an effective strategy to treat cognitive deficits.

Cognitive enhancement by pharmacological interventions is common and self-administration of psychostimulants such as caffeine, nicotine, and amphetamines can improve cognitive performance. However use of these stimulants may not be optimal, they have side effects and risks, and they do not improve all cognitive, memory and learning problems. More effective and specific strategies are needed. Although there are many causes of cognitive, memory and learning problems one strategy to enhance cognitive performance would be to target the key mechanisms that could have a positive effect on cognitive function, such as promoting synapse and dendritic spine structure, function, formation, and regeneration.

In the synaptic context, the actin cytoskeleton is a crucial element in the process of synaptic remodeling and to maintain the dendritic spine and neuronal growth cone architecture driven by synaptic activity. Indeed, neuronal growth cone and dendritic spine shape and synaptic strength are precisely controlled in order to convert short-term alterations of synaptic strength into long-lasting changes that arise from stable structural modifications. These functional and structural modifications are considered the biological basis of memory and learning. Synaptic remodeling involves a number of processes that may be considered as potential targets for cognitive therapies. These processes are highly regulated by signal transduction mechanisms and protein phosphorylation/dephosphorylation, such as occur with cofilin-actin modulation of the cytoarchitecture. The coordination of these complex processes is key to optimized cognitive function.

Cellular stress mediators (inflammation, free radicals, excitotoxic levels of glutamate, mitochondrial inhibitors (e.g., antimycin A), peroxide, NO, Aβ (amyloid) peptides, and proinflammatory cytokines) impair the main functional units of the brain, synapses, and negatively affect their capability to store memories and process information. These results have prompted the idea that improvements in cognition in normal conditions may be achieved by promoting synapse formation. Conditions of memory loss, impaired learning and speed of information processing, as well as loss of motor function after injury are principally conditions of synaptic failure, i.e., a "synaptopathy". Diverse stress mediators, even diets or epigenetic changes, whether apparent or not, can induce the rapid formation of transient or persistent rod-like inclusions containing actin-depolymerizing factor (ADF) /cofilin and actin in axons and dendrites of neurons to cause loss of synapses. The significance of the finding is that rod formation provides a target for intervention to promote synapses because it is correlated with loss of cortical synapses, a cause of lower scores on the Mini-Mental State cognitive examination that become more evident over time. Cognitive impairment in general, by other causes, natural or disease related, such as after brain injury or stroke, or neuropathies are also conditions of synaptic failure and inability to establish functional neuronal connections. Because of its many roles in synaptic function and remodeling, actin dynamics regulation is an attractive target for memory enhancement strategies.

Three factors essential for synapse degeneration are: elevated levels of activated cofilin, elevated levels of ADP-actin, and a highly oxidative environment. Energetically stressed neurons usually meet all three requirements, which are not specifically disease associated. Formation of phosphorylated actin/cofilin rods is an early neuronal pathology that is induced by many causes. When cofilin activation is persistent it leads to synapse loss that precedes neurodegeneration. In cultured neurons death occurs in a few days once cofilin forms rods. The degree and extent to which functioning synapses may be recovered are largely unknown. How pathways of synapse loss relate to synapse reversal is also unclear. We have uncovered specific peptides that act through select chemokine receptors and appear to control the balance between synapse loss and synapse regeneration. The peptides regulate the activation of cellular proteins that control the neuronal cytoarchitecture that gives rise to growth cone structures that contribute to functioning synapses. Reversing synapse loss to recover function is a distinct benefit of the invention over psychostimulants to enhance performance or merely slowing or stopping synapse loss as such loss may proceed unnoticed for decades. Loss of functional neurons is considered irrecoverable, although even that notion seems to be slowly changing. Promoting synapse formation to improve cognition or overcome any synapse loss is the more desirable effect for patient benefit.

More recent advances have begun to illuminate a new method for improving cognition by supporting synapse formation. As explained in detail below, recent discovery has provided new insights into neuronal growth and regeneration mechanisms which suggests that conditions of synapse loss can be now treated by promoting neuro-regeneration directly in the neurons by modulating receptors of the innate immune system present on those neurons. The method provides a means for rebalancing away from what actually is a normal process of synapse deconstruction and construction that supports memory consolidation, sometimes called "pruning", but when excessive or chronic is deleterious, towards regeneration, which is beneficial.

The regenerative treatment has advantages over a psychostimulant or a solely synapse loss preventative. For one, psychostimulants would not lead to durable benefits, while a preventative treatment would only suggest effectiveness in preventing functional losses from progressing. A regenerative method can be reasonably expected to improve normal cognition and in circumstances of synapse loss, such as after brain injury, neuropathies, or dementias to enhance cognition, memory, and learning.

Neuroinflammation, which involves interactions between neurons and components of the innate immune system, like cytokines, chemokines, and microglial cell activity, has been targeted in many preclinical and clinical studies, subsequent clinical trials have largely failed to enhance cognition. A reason is the complexity and multitude of the injury mechanisms which needs either a combination therapy or targeting an early shared disease-causing mechanism. Many actions beyond inflammation control the balance between synapse loss and formation. Blocking inflammation per se does not seem enough to deliver the hope for clinical benefits. Loss of synapses occurs early in many conditions, not necessarily inflammatory, that when left unimpeded will eventually manifest as a cognitive impairment. Currently, early intervention seeks to stop disease progression and worsening, before loss of funtion, cognitive or motor are examples, significantly impairs a person's quality of life, a limited goal. For this reason we sought to refocus efforts to deliver treatments to improve cognition in normal daily living, as well as for synaptopathies and the diseases or conditions that ensue, to deliver greater patient benefit by enhancing cognition or function by promoting formation of functional synapses.

As cognitive impairments are typically not evident at early stages both normal and pathological processes that contribute to synapse loss may continue unabated for years, if not decades, until they become apparent. By the time cognitive impairments manifest, the current state of the art suggests it may be too late to effectively intervene, resulting in permanent loss of functions. Currently no treatments exist to reverse synapse loss although such regeneration would be expected to overcome and reverse existing cognitive deficits and bring a person closer to normal function. Thus while preventing synapse loss by blocking inflammation or interrupting pathogenic cofilin-rod formation that leads to synapse loss would deliver benefits of slower or interrupted disease progression, a distinct and more challenging treatment goal is to reverse synapse damage or loss that is incipient or has already advanced.

The invention is expected to deliver the highly elusive and long-sought treatment benefit of improving memory, learning, cognition and function which manifest in many conditions with loss of synapses, particularly in the hippocampus, a site of learning and memory formation. Such an outcome is expected to have benefits in people that experience loss of synapses, spines, and reduced dendritic arborization beyond slowing or stopping cognitive or functional declines. This invention is directed toward restoration of synapses, dendrites, and neurites to improve cognition and memory, as well as restore cognitive function in dementias and motor function in stroke, or reverse Wallerian degeneration in neuropathies that cause chronic pain conditions caused by injury, surgeries, diabetes, or chemotherapeutic agents. The peptides of the invention offer improved benefit by reversing functional decline's that are both incipient and not readily apparent, and those that have become severe enough to be detected in daily living.

The small peptide chemokine antagonist compounds of the invention offer new treatment opportunities for synapse loss in persons in need of synapse regeneration or experiencding a synaptopathy by targeting specific chemokine receptors that control the balance of synapse formation to destruction and so may be useful in reversing synapse loss in both mild cognitive impairments and conditions of more extensive cognitive impairment.

Numerous studies have shown that mild cognitive impairment (MCI), which can also be a prodromal stage of AD, and early AD are characterized predominantly by loss of synapses and connectivity. Synaptic loss in MCI occurs prior to the wide-spread loss of neurons that characterizes mid and later stages of AD. Neuro-AIDS (HAND), COVID19 or other infections of the CNS, indeed any neurodegenerative condition caused by loss of synapses and even non-pathological conditions such as the loss of synapses of normal aging may be helped. There have been no medical therapies developed to promote recovery in the many diverse conditions united by synapse loss, the synaptopathies

The value of chemokine antagonists as interventions that can reverse synaptopathies was hinted at by decades earlier studies by Hill et al (1) which showed protection of dendritic spine morphology, expansion of dendritic arborization and reversal of functional deficits by the peptide Dala1-peptide T-amide ("DAPTA"), a non-oral analog of the peptides of this invention, in an animal model of HIV cognitive decline. The findings were not well understood at the time as chemokine receptors had not yet been discovered. DAPTA was able to reverse cognitive deficits in HIV-associated neurocognitive disease (HAND) phase 2 human trials and brain scans and the effects were later understood to be mediated by a cluster of HIV entry chemokine receptors (CCR2/CCR5/CCR8/ CXCR4) that were the targets of DAPTA actions. Further attention to the role of CCR5 as a suppressor for cortical plasticity and hippocampal memory and learning came from studies of Zhou, 2016 (2) who showed that in normal mice overexpression of CCR5 caused memory deficits, that could be prevented by CCR5 "knockout", or deletion. In 2019 Joy et al. (3) made the unexpected discovery that CCR5 was a therapeutic target for recovery after stroke and traumatic brain injury. Knockdown of CCR5, or pharmacologic blockade with an antagonist maraviroc, was associated with preservation of dendritic spines and new patterns of neurite cortical projections. The HIV chemokine entry receptor CXCR4 was also identified as having negative effect on recovery after traumatic brain injury as its antagonist AMD3100 improved recovery. Both long-term hippocampal slice cultures from adult rodent brains and dissociated neuronal cultures from mice, show CXCR4 and CCR5 as two important chemokine receptors that when activated lead to synapse loss and cognitive impairments. Other results have implicated CCR2 and CCR8 in memory dysfunctions identifying a cluster of HIV entry receptors which the peptides of the invention target as treatment targets.

Collectively these studies encouraged our investigations into the benefits of a multi-chemokine receptor antagonist peptide to reverse synapse loss by promoting formation of neuronal growth cones, the organizing structure of a neuron that leads to functional synapses. As activation of multiple chemokine pathways are implicated in improved recovery in brain injury, a better outcome is predicted by an agent that can antagonize multiple chemokine receptors to reverse loss of synapses and dendritic spines generally.

### BRIEF DESCRIPTION OF THE DRAWINGS

Fig. 1A illustrates the activity of four representative peptides of the class to block chemokine receptor 2 (CCR2).
Fig. 1B illustrates the activity of several all-D penta-peptides to block chemokine receptor 2 (CCR2).
Fig 2A illustrates direct neuronal activation of the chemokine receptors CXCR4 or CCR5 by the HIV envelope protein gp120 that leads to synapse loss by cofilin-rod formation.
Fig 2B illustrates that cofilin-rod formation, the early pathology causing synapse loss, is caused by the AD associated peptide beta-amyloid (Aβ-trimer/dimer) and can be prevented by blocking neuronal CCR5 with the specific antagonist maraviroc.

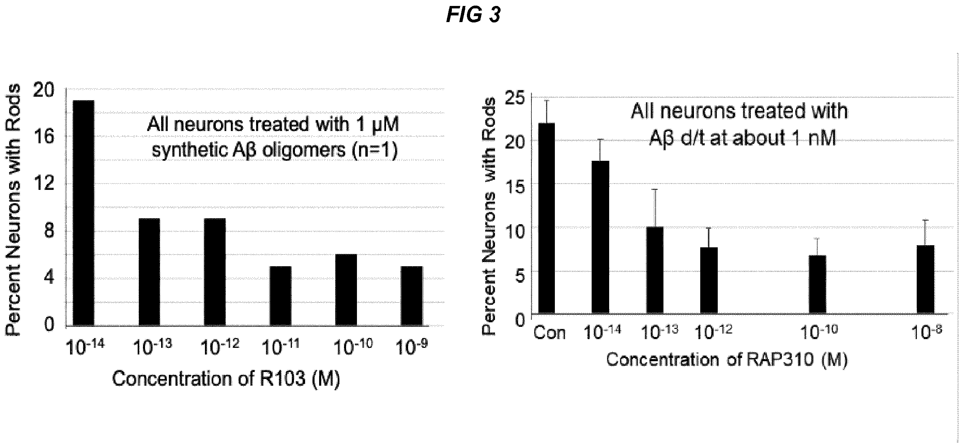

Fig. 3 illustrates the dose response of the multi-chemokine receptor antagonist peptides (RAP-103 (all-D-TTNYT) or RAP-310 (all-D-ASTTTNYT) to prevent Aβ-trimer/dimer pathology causing synapse loss.

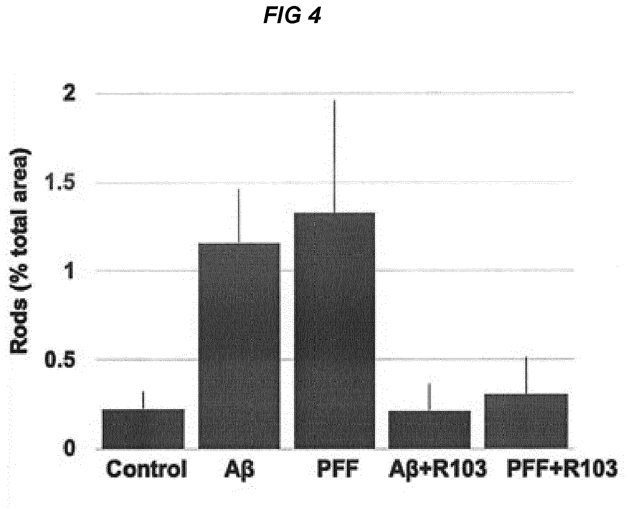

Fig. 4 illustrates that the multi-chemokine receptor antagonist RAP-103 (R103) blocks synapse damaging effects caused by amyloid beta (Aβ) and pre-formed fibrils (PPF) of alpha-synuclein.
Fig. 5 illustrates the ability of amyloid beta (Aβ) trimer to cause pathology (rod formation) causing synapse loss (Left, arrows). Adding RAP-103 (R103) suppresses cofilin activation into rods and shifts to formation of growth cones for synapse regeneration.

In particular embodiments, the invention relates to the treatment of diverse synaptopathies and their associated neurodegenerative conditions which include aging, Alzheimer's Disease, Huntington's Disease and other conditions such as Parkinson's Disease, type 2 diabetes, HIV-associated neurocognitive disorder (HAND), and functional deficits after injury, stroke or trauma , or viral and other encephalopathies which are associated with loss of synapses. The invention also relates to pharmaceutical compositions useful in such treatment and/or reversal of synapse and dendritic spine loss and to certain orally active peptides per se.

Persistent exposure of neurons to soluble β-amyloid peptide dimer/trimers (Aβd/t) (AD), αSynuclein fibrils (PFF), HIV envelope gp120 protein (HAND) or proinflammatory cytokines (TNFα, IL-1β, IL-6) disrupt synaptic function and result in synapse loss. Synapse loss, whether caused by neuroinflammation, oxidative stress, and energetic stress, injury, trauma, bacteria/viruses or other infective agents, cytotoxic drug therapy or radiotherapy underlies many neurodegenerative conditions which the peptides of the present invention may reverse.

Our therapeutic approach seeks to promote synapse formation which has the benefit to reverse synapse loss by blocking activation of neuronal chemokine receptors, a previously unidentified activity of the subject peptides. Targeting neuronal chemokine receptors to promote synapse and growth cone formation with the peptides of the invention to improve memory and cognition is a previously unknown and unexpected treatment advancement and is distinct from prior art whose treatment focus has been to block innate immune inflammation, via microglia and their secreted cytokines to prevent killing of neurons, or to disrupt formation of cofilin-rods that leads to synapse loss just before neurons die. In order to separate out the distinct effects of chemokine receptor blockade we conducted experiments on pure cultures of neurons, compared to slices of brain tissue or whole animals, in the absence of immune cells or cytokines. Distinct from the reports of chemokine blockade in intact animals or CNS tissue slices showing activation of microglia and cytokines, by NKkB pathways, our results point to a previously unrecognized direct effect of blocking neuronal chemokine receptors. We observed chemokine receptor blockade as the means to shift neurons away from a stress response that leads to synapse loss and provoke their regeneration via growth cone formation that drives neurite extension, restores synapses, their connectivity and function in patients who suffer from synapse loss.

The chemokine antagonist peptides of the present invention can block multiple neuronal chemokine receptors at pM doses, an advantage over single receptor antagonists, especially as multiple chemokine receptors seem relevant to synapse recovery. They efficiently and quickly enter the brain by oral and parenteral routes of administration, and due to their protection from proteolysis persist in the CNS at therapeutic doses for periods of time long enough to establish benefits. All of these distinct features combine to deliver an outcome that enables a treatment use in conditions of synapse loss, the synaptopathies, that restores synaptic connectivity to reverse deficits and promote functional recoveries. The invention relates to pharmaceutical compositions useful in such treatment and/or reversal of synapse loss and neurogegeneration and to certain active peptides per se.

The peptides can be used in pharmaceutical compositions and compositions of matter for treating and preventing any disease or condition caused by an organism, compound or immune dysfunction that results in persistent or pathological cofilin-actin rod formation associated with loss of synapses.

The peptides may be administered orally, bucally, parenterally, topically, rectally, vaginally, by intranasal inhalation spray, by intrapulmonary inhalation or in other ways. In particular, the peptides according to the invention may be formulated for oral use, for inhalation with spray or powder, for injection (for example subcutaneous, intramuscular, intravenous, intra-articular or intra-cisternal injection), or for infusion and may be presented in unit dose form in ampoules or tablets or in multidose vials or other containers with an added preservative.

The compositions may take such forms as suspensions, solutions, or emulsions and may contain formulation agents such as suspending, stabilizing and/or dispersing agents. Alternatively, the active ingredient may be in powder and/or lyophilized form for direct administration or for constitution with a suitable vehicle (e.g. sterile, pyrogen-free water, normal saline or 5% dextrose) before use. The pharmaceutical compositions containing peptides(s) may also contain other active ingredients such as antimicrobial agents, or preservatives.

The compositions may contain from 0.001-99% (w/v or, preferably, w/w) of the active material. The preferred administration is an oral pill or capsule. The compositions are administered in therapeutically or prophylactic effective does, i.e. 0.1 to 500 mg of peptide per day, in particular 5-50 mg per day. Even larger doses may be used as the peptide according to the invention is non-toxic. However, normally ths is not required.

For administration by injection or infusion of the compositions, the daily dosage, as employed for treatment of adults of approximately 70 kg of body weight, will often range from 0.2 mg to 20 mg of active material which may be administered in the form of 1 to 4 doses over each day, The invention may be useful in the prevention or treatment of illness or medical conditions, particularly those involving synaptic loss such as mild cognitive impairments, dementias, pre-frontal dementia, Lewy-Body Disease, Alzheimer's Disease, Parkinson's Disease, HAND, stroke recovery or normal aging.

According to a first aspect of the present invention, there is provided the use of a linear peptide of General Formula 1 wherein all amino acids are in the D- stereoisomeric configuration:

A-B-C-D-E-F-G-H. General Formula 1)
wherein
A is Ala, or absent,
B is Ser, Thr or absent,
C is Ser, Thr or absent,
D is Ser, Thr, Asn, Glu, Arg, Ile, Leu,
E is Ser, Thr, Asp, Asn,
F is Thr, Ser, Asn, Arg, Gln, Lys, Trp,
G is Tyr,
H is Thr, Ser, Arg, Gly.

All of the amino acids referred to in General Formula 1 will be in the D-stereoisomeric configuration and candidates for H may be esterified or amidated. The peptide comprises at least 5 amino acids. The all-L amino acid version of a linear peptide of General Formula 1 has been called Peptide T and its modified analog has been called DAPTA in previous studies.

Prefered D-peptides are:
Thr Thr Asn Tyr Thr, (SEQ ID NO:1:)
Ser Ser Thr Tyr Arg, (SEQ ID NO:2:)
Thr Thr Ser Tyr Thr, (SEQ ID NO:3:)
Asn Thr Arg Tyr Arg, (SEQ ID NO:4:)
Ile Asp Asn Tyr Thr, (SEQ ID NO:5:)
Asn Thr Ser Tyr Arg, (SEQ ID NO:6:)
Ile Asn Asn Tyr Thr, (SEQ ID NO:7:)
Asn Thr Ser Tyr Gly, (SEQ ID NO:8:) and
Glu Thr Trp Tyr Ser. (SEQ ID NO:9:).

### METHODS

Aβd/t was isolated from medium of cultured 7PA2 cells, a CHO cell line expressing a form of human APP found in Familial AD. The amount of Aβd/t is quantified by Western blot with standards of synthetic Aβ monomer. Neuronal cultures, fixation, permeabilization, blocking and immunostaining is performed starting with E16.5 mouse hippocampal neurons by common methods in broad use. Assays were performed on 7 and 21 DIV cultures, treated at 6 or 20 DIV with or without drugs (10-14 to 10-10 M for peptides RAP-103 and RAP-310 and 10-11 to 10-7 M maraviroc/AMD3100 with or without ~1 nM Aβd/t. GP120 proteins were obtained from CCR5 tropic strain CM, CXCR4 tropic strain MN, and dual-tropic strain MN. After treatment, cells are fixed and immunostained for cofilin as published and for growth cones with 2G13 antibody and counterstained with DAPI and three coverslips are also stained for the neuronal nuclear antigen NeuN. Analysis will yield the percentage of neurons (NeuN) in total nuclei (DAPI), to obtain rods per neuron.

Images of fixed neurons immunostained for cofilin, growth cones and nuclei captured across the entire coverslip are exported to ImageJ. Macro functions are applied to set local threshold and shape/size segmentation for both the cofilin and growth cone antibodies. Cofilin stained material that is not within neurites is eliminated. Results are reported as rods/nucleus, number of rods per area, rods as percent of total area, or, if NeuN stained or identified morphologically, rods per neuron.

The invention will now be illustrated by the following non-limiting examples. The examples refer to the accompanying drawings.

### Example 1

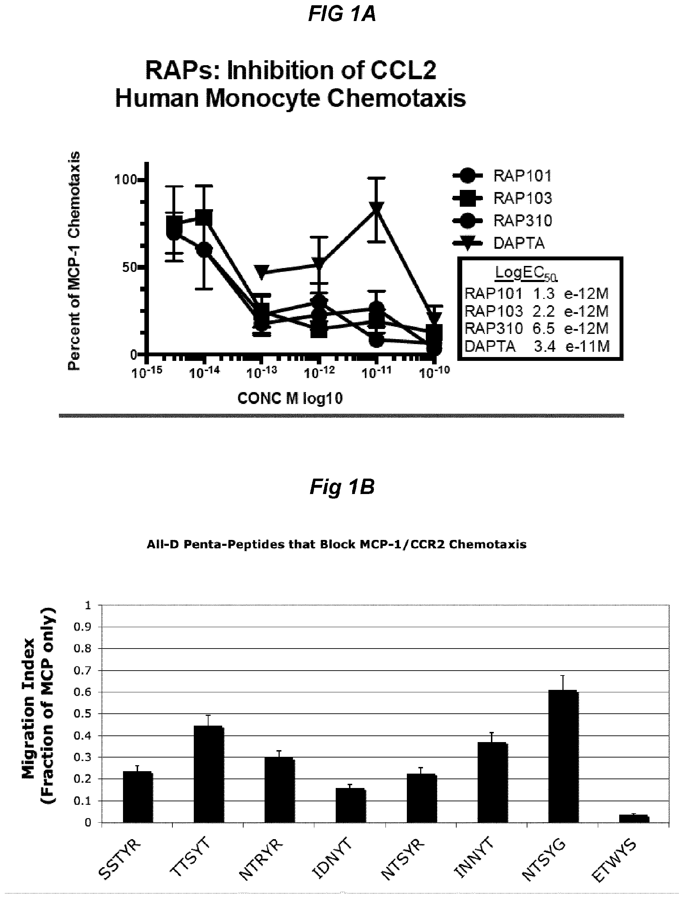

Fig1A illustrates the activity of all-D compared to mostly L-form peptides of formula I. It is shown that three related peptides of general formula I, that have identical primary sequence or that share partial sequence, differing only in enantiomeric form, block CCL2 chemotaxis. The peptides function as chemokine receptor antagonists of both CCR2 and CCR5 (4). Fig 1B shows the general nature of the finding in which eight additional all-D-amino acid pentapeptides also block the CCL1/CCR2 interaction.

### Example 2

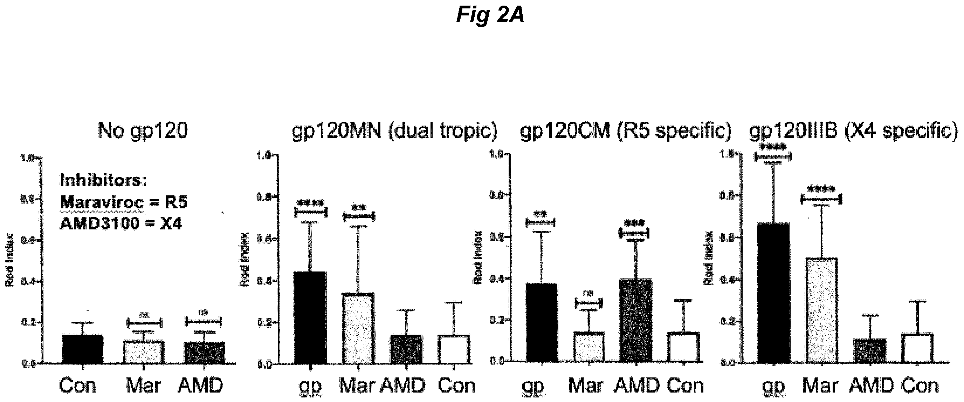

Illustrates that neurons express CCR5 and CXCR4 receptors that control synapse regeneration. Fig 2A shows activation of the chemokine receptors CXCR4 or CCR5 by the HIV envelope protein gp120 in cultured mouse hippocampal neurons devoid of added immune cells, microglia, cytokines or other inflammatory mediators, confirming the relevance of direct neuronal chemokine receptors to synapse loss and cognitive impairments. These chemokine receptor mediated effects of HIV gp120 lead to the cognitive manifestations of neuro-AIDS (HAND), by loss of synapses (shown by Hill et al, (1)) which can therefore be reversed by specific chemokine receptor antagonists maraviroc (Mar, CCR5) or AMD3100 (AMD, CXC4).

Fig 2A also shows that neurons treated with gp120 form cofilin-rods an early transition to synapse destruction, through either CXCR4 or CCR5 receptors as the different gp120 tropic ( CCR5 vs CXCR4) forms are inhibited by their receptor-specific antagonists, Maraviroc (CCR5) and AMD3100 (CXCR4).

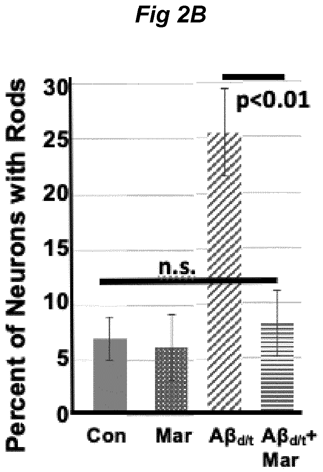

Fig 2B shows that an early pathology of synapse loss, induced by the AD associated peptide beta-amyloid (Aβ-trimer/dimer) can be reversed and synapse regeneration pathways enabled by blocking neuronal CCR5 with maraviroc identifying neuronal chemokine receptors as important in a different (2A, HIV compared to 2B, AD) condition in need of synapse regeneration.

### Example 3

Illustrates the ability of the multi-chemokine receptor (CCR2/CCR5/CCR8/CXCR4) antagonists RAP-103 (all-D-TTNYT) and RAP-310 (all-D-ASTTTNYT) at pM and lower concentrations block Aß and TNF 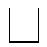 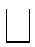 (not shown) early pathology of synapse destruction, formation of cofilin rods in cultured mouse hippocampal neurons. Synapse regeneration cannot occur in the context of activated cofilin-actin rods and the results indicate that the multi-chemokine receptor antagonist peptides shift the neuronal state toward regeneration by reducing the percent of neurons expressing cofiln-rods. Therefore blocking chemokine receptors CCR2/CCR5/CCR8 or CXCR4 is a specific method to block an early AD pathology and reverse synapse loss with the peptides of the invention. Blocking multiple chemokine receptors implicated in synapse loss is an unexpected advantage of the current all-D-peptides of the inventions as they may provide better protection than targeting single chemokine receptors.

### Example 4

Illustrates the ability of the multi-chemokine receptor antagonist RAP-103 to block an early neuronal pathology that results in synapse damage or loss caused by both amyloid beta (Aβ) and pre-formed fibrils (PPF) of alpha-synuclein, the mediators of synapse loss in Alzheimer's and Parkinson's Diseases respectively. The effect of RAP-103 is to shift the neuronal state toward synapse regeneration by reducing the area within the neurons occupied by cofilin-rods.

### Example 5

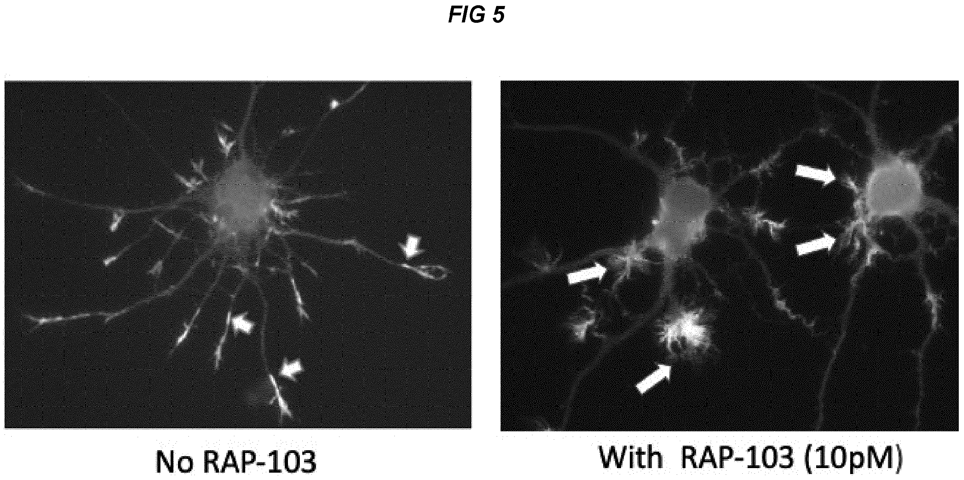

The following experiments illustrate the ability of RAP-103 to promote growth cone formation, a synapse regenerating action. Amyloid beta (Aβ) trimer was added to pure cultures of mouse hippocampal neurons, devoid of immune cells, microglia, and their secreted inflammatory mediators to evaluate actin dynamics and cofilin-rod formation. In Fig 5 left panel, (No RAP-103) synapse pathology (arrows identify immuno-stained rods) is demonstrated for an AD relevant system. Nearly all of the dendrites, especially at their extensions, show rod formation. These neurons will die by day 3. The relevance is that rod pathology leads to synapse loss in people that correlates with dementia. The example further shows (Fig 5, Right) the unexpected effect of RAP-103 to shift neurons from a synapse degenerative state (rods) to a synapse regenerative state (no rods, but now expressing growth cones, stained with 2G13 antibody). Note how in Fig 5, Right, the cofilin rod-like structures at the ends of dendrites are eliminated and replaced with busy growth cone like structures distributed along the entire length of the dendritic arbor as the dominant phenotype. RAP-103 shifts neurons from a state of synapse collapse and loss, to growth cone expansion and regeneration of synapses.

The relevance is that growth cones comprise the growing tip of an axon that seeks out and forms synaptic contacts, and such structures are an aspect of dendritic spine formation, the sites of synapse formation. Blocking chemokine receptors seems to control the bifurcation of a series of complex pathways in actin-cofilin dynamics from synapse destruction and loss to synapse preservation, reconstitution, and regeneration. The results are consistent with other reports of CCR5 blockade as a means to promote synapse formation (3). The significance is that synapse loss underlies all neurodegeneration and causes many conditions and diseases with few to no treatments. The invention offers a novel, significant and unique treatment intervention for broad uses, including non-pathological conditions of synaptopathy, as in normal aging. While the inventors do not claim to know the mechanism of action with certainty we offer our current model to help explain our data that shows two effects caused by the peptides of the invention. The first is blocking cofilin-rod formation with the additional commensurate effect of stimulating growth cone formation. Both features are better than either alone and may represent a unique neuronal regenerative phenotype.

### Literature Cited

1. Hill J, Mervis R, Avidor R, Moody T, Brenneman D. HIV envelope protein-induced neuronal damage and retardation of behavioral development in rat neonates. Brain Res. 1993;603(2):222-33.
2. Zhou M, Greenhill S, Huang S, Silva TK, Sano Y, Wu S, Cai Y, Nagaoka Y, Sehgal M, Cai DJ, Lee YS, Fox K, Silva AJ. CCR5 is a suppressor for cortical plasticity and hippocampal learning and memory. Elife. 2016;5. Epub 2016/12/21. doi: 10.7554/eLife.20985. PubMed PMID: 27996938; PMCID: PMC5213777.
3. Joy MT, Ben Assayag E, Shabashov-Stone D, Liraz-Zaltsman S, Mazzitelli J, Arenas M, Abduljawad N, Kliper E, Korczyn AD, Thareja NS, Kesner EL, Zhou M, Huang S, Silva TK, Katz N, Bornstein NM, Silva AJ, Shohami E, Carmichael ST. CCR5 Is a Therapeutic Target for Recovery after Stroke and Traumatic Brain Injury. Cell. 2019;176(5):1143-57 e13. Epub 2019/02/23. doi: 10.1016/j.cell.2019.01.044. PubMed PMID: 30794775.
4. Padi S, Shi X, Zhao Y, Ruff M, Baichoo N, Pert C, Zhang J. Attenuation of rodent neuropathic pain by an orally active peptide, RAP-103, which potently blocks CCR2- and CCR5-mediated monocyte chemotaxis and inflammation. Pain. 2012;153(1):95-106. doi: S0304-3959(11)00579-3 [pii] 10.1016/j.pain.2011.09.022. PubMed PMID: 22033364; PMCID: 22033364.

The following paragraphs describe aspects of the invention:
1. A method of improving cognitive function in a person by enhancing synapse and dendritic spine formation comprising the steps of:
   preparing a composition comprising a D peptide multi-chemokine receptor antagonist in a pharmaceutically acceptable carrier,
   said D peptide further comprises the general structure: A-B-C-D-E-F-G-H in which:
      A is Ala, or absent,
      B is Ser, Thr or absent,
      C is Ser, Thr or absent,
      D is Ser, Thr, Asn, Glu, Arg, Ile, Leu,
      E is Ser, Thr, Asp, Asn,
      F is Thr, Ser, Asn, Arg, Gin, Lys, Trp,
      G is Tyr, and
      H is Thr, Ser, Arg, Gly,
   wherein said D peptide further comprises all amino acids being in the D stereoisomeric configuration, and
   administering said composition to the person in a therapeutically effective dose, wherein said composition acts to prevent formation of actin/cofilin rods which enhances formation of synapses and dendritic spines and thus improves cognitive function in the person.
2. The method as defined in paragraph 1 wherein the person further comprises a person experiencing symptoms of synaptic and dentritic spine loss.
3. The method as defined in paragraph 2 wherein the symptoms of synaptic and dentritic spine loss are selected from the group consisting of: age related cognitive function loss, mild cognitive impairment, dementia related cognitive function loss, brain trauma related cognitive function loss, neuropathies.
4. The method as defined in paragraph 2 wherein the symptoms of synapse and dentritic spine loss are selected from the group consisting of: Alzheimer's Disease, Pre-frontal dementia, Huntington's Disease, synucleinopathy, Parkinson's Disease, Lewy Body Disease, neuro-AIDS/HAND, demyelinating disease, HTLV-1-associated myelopathy (HAM), multiple sclerosis (MS), amyotrophic lateral sclerosis, cognitive decline in diabetes, normal aging, or the pathological neurological symptoms and behaviors occurring after brain injury, stroke, diabetes, neuropathy, encephalopathy and viral encephalopathy.
5. The method as defined in paragraph 1 wherein administering said composition to the patient is selected from the group consisting of administrating: orally, buccally, parenterally, topically, rectally, vaginally, by intranasal inhalation spray, by intrapulmonary inhalation.
6. The method as defined in paragraph 1 further comprising,
   said D peptide is at most twenty (20) D amino acid residues in length and contains five contiguous D amino acid residues that have a sequence selected from the group consisting of:
   Thr Thr Asn Tyr Thr, (SEQ ID NO:1:)
   Ser Ser Thr Tyr Arg, (SEQ ID NO:2:)
   Thr Thr Ser Tyr Thr, (SEQ ID NO:3:)
   Asn Thr Arg Tyr Arg, (SEQ ID NO:4:)
   Ile Asp Asn Tyr Thr, (SEQ ID NO:5:)
   Asn Thr Ser Tyr Arg, (SEQ ID NO:6:)
   Ile Asn Asn Tyr Thr, (SEQ ID NO:7:)
   Asn Thr Ser Tyr Gly, (SEQ ID NO:8:) and
   Glu Thr Trp Tyr Ser. (SEQ ID NO:9:).
7. The method as defined in paragraph 6 further comprising,
   said D peptide derivative is at most twelve (12) D amino acid residues in length.
8. The method as defined in paragraph 6 further comprising,
   said D peptide derivative is at most eight (8) D amino acid residues in length.
9. The method as defined in paragraph 6 further
   comprising,said D peptide is five (5) D amino acid residues in length.
10. A method of treating loss of synapses and dendritic spines in a person experiencing symptoms of synapses and dendritic spine loss comprising the steps of:
   preparing a composition comprising a peptide analog and a pharmaceutically acceptable carrier,
   said peptide analog is [D-Ala1]-Ser-Thr-Thr-Thr-Asn-Tyr-Thr-NH2 in which the first amino acid is a D stereoisomer and the remaining amino acids are L stereoisomers and the N-terminal amino acid is an amide, and
   administering said peptide analog to the patient in a therapeutically effective dose,
   wherein said composition acts to treat the loss of synapses and dendritic spines in the patient.
11. The method as defined in paragraph 10 wherein symptoms of synapses and dendritic spines loss are selected from the group consisting of Alzheimer's Disease, Huntington's Disease, synucleinopathy, Parkinson's Disease, demyelinating disease, HTLV-1-associated myelopathy (HAM), multiple sclerosis (MS), amyotrophic lateral sclerosis, pathological neurological symptoms after injury or trauma, encephalopathy and viral encephalopathy.

The following paragraphs also describe aspects of the invention:
Paragraph 1. A composition comprising a D peptide multi-chemokine receptor antagonist in a pharmaceutically acceptable carrier for use in a method of improving cognitive function in a person by enhancing synapse and dendritic spine formation, wherein
   said D peptide further comprises the general structure: A-B-C-D-E-F-G-H in which: A is Ala, or absent,
   B is Ser, Thr or absent, C is Ser, Thr or absent,
   D is Ser, Thr, Asn, Glu, Arg, Ile, Leu, E is Ser, Thr, Asp, Asn,
   F is Thr, Ser, Asn, Arg, Gin, Lys, Trp, G is Tyr, and
   H is Thr, Ser, Arg, Gly;
   wherein said D peptide further comprises all amino acids being in the D stereoisomeric configuration except when Gly is present and Gly is achiral; and wherein
   said composition acts to prevent formation of actin/cofilin rods which enhances formation of synapses and dendritic spines and thus improves cognitive function in the person.
Paragraph 2. A composition for use according to paragraph 1 wherein the person is experiencing symptoms of synaptic and dentritic spine loss. Paragraph 3. A composition for use according to paragraph 2 wherein the symptoms of synaptic and dentritic spine loss are selected from the group consisting of: age related cognitive function loss, mild cognitive impairment, dementia related cognitive function loss, brain trauma related cognitive function loss, neuropathies.
Paragraph 4. A composition for use according to paragraph 2 wherein the symptoms of synapse and dentritic spine loss are selected from the group consisting of: Alzheimer's Disease, Pre-frontal dementia, Huntington's Disease, synucleinopathy, Parkinson's Disease, Lewy Body Disease, neuro-AIDS/HAND, demyelinating disease, HTLV-1-associated myelopathy (HAM), multiple sclerosis (MS), amyotrophic lateral sclerosis, cognitive decline in diabetes, normal aging, or the pathological neurological symptoms and behaviors occurring after brain injury, stroke, diabetes, neuropathy, encephalopathy and viral encephalopathy.
Paragraph 5. A composition for use according to paragraph 1 wherein administering said composition to the patient is selected from the group consisting of administrating: orally, buccally, parenterally, topically, rectally, vaginally, by intranasal inhalation spray, by intrapulmonary inhalation.
Paragraph 6. A composition for use according to paragraph 1 further comprising,
   said D peptide is at most twenty (20) D amino acid residues in length and contains five contiguous D amino acid residues except when Gly is present and Gly is achiral that have a sequence selected from the group consisting of:
   Thr Thr Asn Tyr Thr, (SEQ ID NO:1:) Ser Ser Thr Tyr Arg, (SEQ ID NO:2:) Thr Thr Ser Tyr Thr, (SEQ ID NO:3:) Asn Thr Arg Tyr Arg, (SEQ ID NO:4:) Ile Asp Asn Tyr Thr, (SEQ ID NO:5:) Asn Thr Ser Tyr Arg, (SEQ ID NO:6:) Ile Asn Asn Tyr Thr, (SEQ ID NO:7:) Asn Thr Ser Tyr Gly, (SEQ ID NO:8:) and Glu Thr Trp Tyr Ser. (SEQ ID NO:9:).
Paragraph 7. A composition for use according to paragraph 6 further comprising,
   said D peptide derivative is at most twelve (12) D amino acid residues in length.
Paragraph 8. A composition for use according to paragraph 6 further comprising,
   said D peptide derivative is at most eight (8) D amino acid residues in length.
Paragraph 9. A composition for use according to paragraph 6 further comprising, said D peptide is five (5) D amino acid residues in length.
Paragraph 10. A composition comprising a peptide analog and a pharmaceutically acceptable carrier for use in a method of treating loss of synapses and dendritic spines in a person experiencing one or more symptoms of synapses and dendritic spine loss wherein said peptide analog is [D-Ala1]-Ser-Thr-Thr-Thr-Asn-Tyr-Thr-NH2
   in which the first amino acid is a D stereoisomer and the remaining amino acids are L stereoisomers and the N-terminal amino acid is an amide, and wherein said composition acts to treat the loss of synapses and dendritic spines in the patient.
Paragraph 11. A composition for use according to paragraph 10 wherein symptoms of synapses and dendritic spines loss are selected from the group consisting of Alzheimer's Disease, Huntington's Disease, synucleinopathy, Parkinson's Disease, demyelinating disease, HTLV-1-associated myelopathy (HAM), multiple sclerosis (MS), amyotrophic lateral sclerosis, pathological neurological symptoms after injury or trauma, encephalopathy and viral encephalopathy.

The present invention relates, broadly, to the improvement of cognition by methods to shift neurons from a state or condition of loss of synapses and dendritic spines to one of formation/regeneration of synapses and dendritic spines. A novel mechanism appears to control a neuronal stress response and improve memory and learning by rebalancing synapse and dendritic spine dynamics to favor their formation. Methods are disclosed to enhance the number of functioning synapses and dendritic spines which is expected to improve overall cognitive function.

## Claims

1. A composition comprising a D peptide and a pharmaceutically acceptable carrier for use in treating a disease selected from: Alzheimer's Disease, Lewy Body Disease, or Parkinson's disease; wherein said D peptide further comprises the general structure: A-B-C-D-E-F-G-H in which:
A is Ala, or absent,
B is Ser, Thr or absent,
C is Ser, Thr or absent,
D is Ser, Thr, Asn, Glu, Arg, Ile, Leu,
E is Ser, Thr, Asp, Asn,
F is Thr, Ser, Asn, Arg, Gin, Lys, Trp,
G is Tyr, and
H is Thr, Ser, Arg, Gly;
wherein said D peptide comprises all stereoisomeric amino acids in the D stereoisomeric configuration.

2. The composition for use according to claim 1, wherein the disease comprises Alzheimer's Disease.

3. The composition for use according to claim 2, wherein the disease comprises Lewy Body Disease.

4. The composition for use according to claim 2, wherein the disease comprises Parkinson's disease.

5. The composition for use according to claim 1, wherein said composition acts to prevent formation of actin/cofilin rods in an in vitro assay.

6. The composition for use according to claim 1, wherein said D peptide is at most twenty (20) amino acid residues and comprises a sequence selected from the group consisting of:
Thr Thr Asn Tyr Thr, (SEQ ID NO:1:)
Ser Ser Thr Tyr Arg, (SEQ ID NO:2:)
Thr Thr Ser Tyr Thr, (SEQ ID NO:3:)
Asn Thr Arg Tyr Arg, (SEQ ID NO:4:)
Ile Asp Asn Tyr Thr, (SEQ ID NO:5:)
Asn Thr Ser Tyr Arg, (SEQ ID NO:6:)
Ile Asn Asn Tyr Thr, (SEQ ID NO:7:)
Asn Thr Ser Tyr Gly, (SEQ ID NO:8:) and
Glu Thr Trp Tyr Ser, (SEQ ID NO:9:), wherein said D peptide comprises all stereoisomeric amino acids in the D stereoisomeric configuration.

7. The composition for use according to claim 6, wherein said D peptide derivative is at most twelve (12) D amino acid residues in length.

8. The composition for use according to claim 6, wherein said D peptide derivative is at most eight (8) D amino acid residues in length.

9. The composition for use according to claim 6, wherein said D peptide is five (5) D amino acid residues in length.

10. A composition comprising a peptide analog and a pharmaceutically acceptable carrier for use in treating Alzheimer's Disease wherein said peptide analog is [D-Ala1]-Ser-Thr-Thr-Thr-Asn-Tyr-Thr-NH2, and wherein
(i) the first amino acid is a D stereoisomer;
(ii) the remaining amino acids are L stereoisomers; and
(iii) the N-terminal amino acid is an amide.

11. The composition for use according to claim 1, wherein the composition stimulates growth cone formation in an in vitro assay.

12. The composition for use according to claim 1, wherein the composition is in the form of a solution.

13. The composition for use according to claim 1, wherein the composition comprises 0.1 to 500 mg of the D-peptide.

14. The composition for use according to claim 1, wherein the composition enhances formation of dendritic spines in an in vitro assay.

15. The composition for use according to claim 1, wherein the composition is in the form of a pill.


SEQUENCE LISTING

<110>  Creative Bio-Peptides Inc.

<120>  ORAL PEPTIDE ANTAGONISTS OF MULTIPLE CHEMOKINE RECEPTORS FOR
       REVERSING LOSS OF SYNAPSES AND DENDRITIC SPINES

<130>  P105822EP01

<140>  17170790
<141>  2021-02-08

<150>  62970867
<151>  2020-02-06

<160>  9

<170>  PatentIn version 3.5

<210>  1
<211>  5
<212>  PRT
<213>  Human immunodeficiency virus

<400>  1

Thr Thr Asn Tyr Thr
1               5


<210>  2
<211>  5
<212>  PRT
<213>  Human immunodeficiency virus

<400>  2

Ser Ser Thr Tyr Arg
1               5


<210>  3
<211>  5
<212>  PRT
<213>  Human immunodeficiency virus

<400>  3

Thr Thr Ser Tyr Thr
1               5


<210>  4
<211>  5
<212>  PRT
<213>  Human immunodeficiency virus

<400>  4

Asn Thr Arg Tyr Arg
1               5

<210>  5
<211>  5
<212>  PRT
<213>  Human immunodeficiency virus

<400>  5

Ile Asp Asn Tyr Thr
1               5

<210>  6
<211>  5
<212>  PRT
<213>  Human immunodeficiency virus

<400>  6

Asn Thr Ser Tyr Arg
1               5

<210>  7
<211>  5
<212>  PRT
<213>  Human immunodeficiency virus

<400>  7

Ile Asn Asn Tyr Thr
1               5

<210>  8
<211>  5
<212>  PRT
<213>  Human immunodeficiency virus

<400>  8

Asn Thr Ser Tyr Gly
1               5

<210>  9
<211>  5
<212>  PRT
<213>  Human immunodeficiency virus

<400>  9

Glu Thr Trp Tyr Ser
1               5